# EUROPÄISCHE PATENTSCHRIFT

(11) **EP 3 946 506 B1**
(45) Veröffentlichungstag und Bekanntmachung des Hinweises auf die Patenterteilung: **24.08.2022**
(21) Anmeldenummer: 21712134.2
(22) Anmeldetag: 12.03.2021
(51) Int. Cl.: A61M 1/16

(54) **SYSTEM ZUR DETEKTION VON BLUT IN EINER DIALYSATSTRÖMUNG EINES DIALYSEGERÄTS**
SYSTEM FOR DETECTION OF BLOOD IN THE DIALYSATE FLOW OF A DIALYSIS MACHINE
SYSTÈME POUR LA DETECTION DE SANG DANS LE DÉBIT DE DIALYSAT D'UNE MACHINE DE DIALYSE

(30) Priorität: 23.03.2020 DE 102020203674
(43) Veröffentlichungstag der Anmeldung: 09.02.2022
(73) Patentinhaber: B. Braun Avitum AG, 34212 Melsungen (DE)
(72) Erfinder: LECKE, Tobias, 36179 Bebra (DE)
(74) Vertreter: Patentanwälte Ruff, Wilhelm, Beier, Dauster & Partner mbB
(86) Internationale Anmeldenummer: PCT/EP2021/056307
(87) Internationale Veröffentlichungsnummer: WO 2021/190954

(56) Entgegenhaltungen:
- DE-A1-102011 008 482
- US-A1- 2016 058 933

## Beschreibung

Die Erfindung betrifft eine Detektionseinrichtung und ein Verfahren zur Detektion von Blut in einer Dialysatströmung eines Dialysegeräts bei einer extrakorporalen Blutbehandlung.

Zur extrakorporalen Blutbehandlung vorgesehene Dialysegeräte weisen einen Dialysator mit einer Blutkammer und einer Dialysatkammer auf, die mittels einer semipermeablen Membran voneinander getrennt sind. Bei der Blutbehandlung wird Blut in einem extrakorporalen Blutkreislauf durch die Blutkammer gefördert. Gleichzeitig wird die Dialysatkammer von einem Dialysat durchströmt, das auch als Dialysierflüssigkeit bezeichnet werden kann. Bei einer versagensbedingten Ruptur der semipermeablen Membran kann Blut aus dem extrakorporalen Blutkreislauf in die Dialysatströmung gelangen. Ein solches Blutleck kann zu schwerwiegenden medizinischen Beeinträchtigungen des zu behandelnden Patienten und zu technischen Fehlern am Dialysegerät führen. Aus diesem Grund sind Dialysegeräte üblicherweise mit einer Detektionseinrichtung versehen, mittels derer Blut in der Dialysatströmung detektiert werden kann.

Eine derartige Detektionseinrichtung ist aus der US 4,181,610 B1 bekannt. Die bekannte Detektionseinrichtung weist eine erste Lichtquelle und eine zweite Lichtquelle auf, die gemeinsam auf einer ersten Seite eines lichtdurchlässigen Fluidleitungskanals zur Fluidleitung der Dialysatströmung angeordnet sind und Licht unterschiedlicher Wellenlänge emittieren. Zudem weist die bekannte Detektionseinrichtung eine Steuereinrichtung auf, die zum abwechselnden Ansteuern der beiden Lichtquellen eingerichtet ist, so dass deren Licht abwechselnd in die Dialysatströmung eingestrahlt wird. Auf einer gegenüberliegenden Seite des Fluidleitungskanals ist ein einziger Detektor angeordnet, der zum Erfassen der durch die Dialysatströmung transmittierten Lichtanteile der beiden Lichtquellen und zum Erzeugen entsprechender Signale eingerichtet ist. Dabei repräsentiert ein erstes Signal die Intensität des erfassten transmittierten Lichtanteils der ersten Lichtquelle. Ein zweites Signal repräsentiert die Intensität des erfassten transmittierten Lichtanteils der zweiten Lichtquelle. Bei einem Blutleck wird das von der ersten Lichtquelle eingestrahlte Licht aufgrund seiner Wellenlänge stärker absorbiert als das von der zweiten Lichtquelle eingestrahlte Licht. Hierdurch kann das Blutleck durch einen Vergleich zwischen dem ersten Signal und dem zweiten Signal detektiert werden. Weiter weist die bekannte Detektionseinrichtung eine Kalibriereinrichtung auf, die zum Kalibrieren der Helligkeiten der beiden Lichtquellen eingerichtet ist. Hierdurch soll vermieden werden, dass betriebsbedingte Helligkeitsschwankungen zwischen den beiden Lichtquellen fälschlicherweise als Blutleck gedeutet werden. US2016058933 offenbart eine Detektionseinrichtung zur Detektion von Blut in dem Dialysat, aufweisend eine Lichtquelle zum Einstrahlen von Licht in die Dialysatströmung, einen den durch die Dialysatströmung transmittierten UV-Lichtanteil erfassenden ersten Detektor und eine Auswerteeinheit, die dazu konfiguriert ist, basierend auf den von dem Detektor erfassenen Daten ein Blutdetektionssignal zu erzeugen. Des weiteren wird darin vorgeschlagen, gestreutes Licht in statt von transmittiertem Licht zu detektieren.

Aufgabe der Erfindung ist es, eine Detektionseinrichtung und ein Verfahren der eingangs genannten Art bereitzustellen, die gegenüber dem Stand der Technik einen vereinfachten Aufbau bzw. eine vereinfachte Durchführung und gleichzeitig eine zuverlässige Detektion von Blut in der Dialysatströmung ermöglichen.

Diese Aufgabe wird durch das Bereitstellen eines erfindungsgemäßen Verfahrens mit den Merkmalen des Anspruchs 1 und einer erfindungsgemäßen Detektionseinrichtung mit den Merkmalen des Anspruchs 5 gelöst.

Das erfindungsgemäße Verfahren umfasst die Schritte: a) Einstrahlen von Licht in die Dialysatströmung; b) Erfassen eines durch die Dialysatströmung hindurch transmittierten Lichtanteils des eingestrahlten Lichts an einem ersten Erfassungsort und Erzeugen eines ersten Signals, das die Intensität des erfassten transmittierten Lichtanteils repräsentiert; c) Erfassen eines in der Dialysatströmung gestreuten Lichtanteils des eingestrahlten Lichts an einem zweiten Erfassungsort und Erzeugen eines zweiten Signals, das die Intensität des erfassten gestreuten Lichtanteils repräsentiert; d) Erzeugen eines Detektionssignals in Abhängigkeit des erzeugten ersten Signals und des erzeugten zweiten Signals. Durch die erfindungsgemäße Lösung kann auf eine aufwändige Kalibrierung der Helligkeit des eingestrahlten Lichts verzichtet werden. Denn etwaige Intensitätsschwankungen des eingestrahlten Lichts wirken sich gleichermaßen auf den transmittierten Lichtanteil und auf den gestreuten Lichtanteil aus. Dementsprechend ändern sich das erste Signal und das zweite Signal bei Intensitätsschwankungen des eingestrahlten Lichts gleichermaßen positiv oder gleichermaßen negativ. Demgegenüber nimmt die Intensität des transmittierten Lichtanteils mit zunehmender Blutkonzentration in der Dialysatströmung ab und gleichzeitig nimmt die Intensität des gestreuten Lichtanteils zu. Dementsprechend ändern sich das erste Signal und das zweite Signal bei einem Blutleck - anders als bei einer Helligkeitsschwankung des eingestrahlten Lichts - gegenläufig. Dies kann beim Erzeugen des Detektionssignals in Abhängigkeit des ersten Signals und des zweiten Signals berücksichtigt werden. Im Ergebnis ermöglicht das erfindungsgemäße Verfahren eine einfache und dennoch zuverlässige Detektion von Blut in der Dialysatströmung, da fehlerhafte Detektionen aufgrund von Helligkeitsschwankungen des eingestrahlten Lichts bei gleichzeitigem Verzicht auf eine diesbezügliche Kalibrierung vermieden werden.

Der Schritt a) umfasst das Einstrahlen des Lichts in die Dialysatströmung. Dabei haben die Erfinder erkannt, dass das Einstrahlen von für den Menschen sichtbarem Licht besonders vorteilhaft ist. Insoweit kann beispielsweise rotes, grünes oder blaues Licht eingestrahlt werden, wobei "rot" und "blau" die Grenzen des sichtbaren Spektrums repräsentieren. Denn hierdurch kann eine Beeinträchtigung der Detektion durch in der Dialysatströmung befindliche harnpflichtige Stoffe vermieden werden. Weiter wurde erkannt, dass das Einstrahlen von blauem Licht Vorteile im Hinblick auf ein besonders sensitives Erfassen des Lichts insbesondere im Schritt c) bietet. Vorzugsweise wird das Licht in einer quer, bevorzugt senkrecht, zu einer Strömungsrichtung der Dialysatströmung orientierten Einstrahlrichtung eingestrahlt. Das Licht kann zeitkontinuierlich und/oder zeitdiskret eingestrahlt werden.

Der Schritt b) umfasst das Erfassen des durch die Dialysatströmung hindurch transmittierten Lichtanteils des eingestrahlten Lichts. Die Intensität des erfassten transmittierten Lichtanteils nimmt bei einem Blutleck ab. Dies aufgrund von Absorptions-, Reflektions- und/oder Streueffekten an den Blutpartikeln des eingeleckten Bluts. Die "Summe" aus Absorption, Streuung und weiteren die Intensität des Lichts mindernden Effekten kann auch als Extinktion bezeichnet werden. Der Schritt b) umfasst zudem das Erzeugen des ersten Signals. Da das erste Signal die Intensität des erfassten transmittierten Lichtanteils repräsentiert, ändert sich das erste Signal, insbesondere dessen Wert, bei einem Blutleck dementsprechend. Vorzugsweise nimmt das erste Signal bei einem Blutleck ab. Mit anderen Worten verringert sich der Wert des ersten Signals bei einem Blutleck über der Zeit. Mit nochmals anderen Worten ist eine zeitliche Änderung des ersten Signals bei einem Blutleck vorzugsweise negativ. Das erste Signal kann zeitdiskret und/oder zeitkontinuierlich erzeugt werden.

Der Schritt c) umfasst das Erfassen des in der Dialysatströmung gestreuten Lichtanteils des eingestrahlten Lichts. Bei einem Blutleck steigt die Konzentration lichtstreuender Blutpartikel in der Dialysatströmung. Dementsprechend nimmt die Intensität des gestreuten Lichtanteils bei einem Blutleck zu. Der Schritt c) umfasst zudem das Erzeugen des zweiten Signals. Da das zweite Signal die Intensität des erfassten gestreuten Lichtanteils repräsentiert, ändert sich das zweite Signal, insbesondere dessen Wert, dementsprechend. Vorzugsweise nimmt das zweite Signal bei einem Blutleck zu. Mit anderen Worten ist eine zeitliche Änderung des zweiten Signals bei einem Blutleck vorzugsweise positiv. Das zweite Signal kann zeitkontinuierlich und/oder zeitdiskret erzeugt werden.

Der Schritt d) umfasst das Erzeugen des Detektionssignals. Das Detektionssignal kann insbesondere ein Signal zum Steuern wenigstens einer Funktion einer Detektionseinrichtung zum Durchführen des Verfahrens, wenigstens einer Funktion des Dialysegeräts und/oder ein von einem Anwender des Verfahrens wahrnehmbares, insbesondere akustisches und/oder optisches, Signal sein. Das Detektionssignal wird in Abhängigkeit des erzeugten ersten Signals und des erzeugten zweiten Signals erzeugt. Hierbei können insbesondere ein Wert, ein Vorzeichen, ein Betrag, eine zeitliche Änderung, eine zeitliche Änderungsrate oder dergleichen des jeweiligen Signals ausgewertet werden.

In Ausgestaltung der Erfindung wird das Detektionssignal dann erzeugt, wenn eine zeitliche Änderung des ersten Signals gegenläufig ist zu einer zeitlichen Änderung des zweiten Signals. Demnach wird das Detektionssignal dann erzeugt, wenn das erste Signal, insbesondere dessen Wert, über der Zeit abnimmt und gleichzeitig das zweite Signal, insbesondere dessen Wert, über der Zeit zunimmt oder umgekehrt. Dies ist eine besonders vorteilhafte Ausgestaltung der Erfindung.

In weiterer Ausgestaltung der Erfindung umfasst das Verfahren die Schritte: e) Emittieren von UV-Licht, wobei das UV-Licht in die Dialysatströmung eingestrahlt und an der Dialysatströmung vorbeigestrahlt wird; f) Erfassen eines durch die Dialysatströmung hindurch transmittierten UV-Lichtanteils des eingestrahlten UV-Lichts an dem ersten Erfassungsort und Erzeugen eines dritten Signals, das die Intensität des erfassten transmittierten UV-Lichtanteils repräsentiert; g) Erfassen des an der Dialysatströmung vorbeigestrahlten UV-Lichts an dem zweiten Erfassungsort und Erzeugen eines vierten Signals, das die Intensität des erfassten vorbeigestrahlten UV-Lichts repräsentiert; h) Ermitteln eines Kt/V-Werts in Abhängigkeit des erzeugten dritten Signals und des erzeugten vierten Signals. Dies ist eine besonders bevorzugte Ausgestaltung der Erfindung. Denn diese Ausgestaltung ermöglicht eine zusätzliche Ermittlung des sogenannten Kt/V-Werts unter einer "ortsgleichen" Erfassung der hierfür erforderlichen UV-Lichtintensitäten. Die Bezeichnung "Kt/V-Wert" oder kurz "Kt-V" ist im Bereich der Medizintechnik als solches bekannt. Der Kt/V-Wert steht in Relation zu einer Harnstoffkonzentration in der Dialysatströmung. Ist eine relative Änderung der Harnstoffkonzentration bekannt, kann der Kt/V-Wert auf Grundlage bekannter chemischer und/oder physikalischer Beziehungen ermittelt werden. Die Änderung der Harnstoffkonzentration steht in Zusammenhang mit einer Änderung des erzeugten dritten Signals. Mit anderen Worten ausgedrückt: Die Harnstoffkonzentration in der Dialysatströmung ist ein Marker für den Fortschritt der extrakorporalen Blutbehandlung; je geringer die Konzentration in der Dialysatströmung, desto geringer die Konzentration im Blut des zu behandelnden Patienten; eine relative Messung dieser Konzentration erlaubt es, den Kt/V-Wert zu bestimmen. Im Ergebnis gestattet der Kt/V-Wert bekanntlich einen Rückschluss auf den Fortschritt der extrakorporalen Blutbehandlung und damit insbesondere auf eine erforderliche Behandlungsdauer. Die zur Ermittlung des Kt/V-Werts erforderlichen Intensitäten des UV-Lichts werden am ersten Erfassungsort und am zweiten Erfassungsort und somit an den Erfassungsorten der Blutleck-Detektion erfasst. Dies gestattet eine maßgebliche Vereinfachung der Durchführung des Verfahrens und ermöglicht gleichzeitig eine maßgebliche Vereinfachung des Aufbaus einer zum Durchführen des Verfahrens eingerichteten Detektionseinrichtung. Vor diesem Hintergrund betrifft diese Ausgestaltung der Erfindung somit ein Verfahren zur Detektion von Blut und einem Toxin, insbesondere Harnstoff, in einer Dialysatströmung eines Dialysegeräts bei einer extrakorporalen Blutbehandlung. Da das Verfahren gemäß dieser Ausgestaltung der Erfindung sowohl zur Detektion von Blut als auch des Toxins vorgesehen ist, kann auch von einer "kombinierten Detektion" gesprochen werden.

Der Schritt e) umfasst das Emittieren von UV-Licht. Das emittierte UV-Licht wird in die Dialysatströmung ein- und an derselben vorbeigestrahlt. Das UV-Licht wird vorzugsweise in einer Einstrahlrichtung in die Dialysatströmung eingestrahlt, die quer, bevorzugt senkrecht, zur Strömungsrichtung der Dialysatströmung orientiert ist. Weiter vorzugsweise werden das UV-Licht und das - gemäß Schritt a) einzustrahlende - Licht in einer gemeinsamen Ebene in die Dialysatströmung eingestrahlt. Das UV-Licht kann zeitkontinuierlich und/oder zeitdiskret emittiert, eingestrahlt und/oder vorbeigestrahlt werden. Vorzugsweise wird das UV-Licht in einer quer, bevorzugt senkrecht, zur Strömungsrichtung der Dialysatströmung orientierten Abstrahlrichtung an der Dialysatströmung vorbeigestrahlt.

Der Schritt f) umfasst das Erfassen des durch die Dialysatströmung hindurch transmittierten UV-Lichtanteils des eingestrahlten UV-Lichts. Mit zunehmender Harnstoffkonzentration nimmt die Intensität des transmittierten UV-Lichtanteils ab. Dies aufgrund von Absorptions-, Reflektions- und/oder Streueffekten. Der Schritt f) umfasst zudem das Erzeugen des dritten Signals. Da dieses die Intensität des erfassten transmittierten UV-Lichtanteils repräsentiert, nimmt das dritte Signal, insbesondere dessen Wert, mit zunehmender Harnstoffkonzentration in der Dialysatströmung ab. Das dritte Signal kann zeitkontinuierlich und/oder zeitdiskret erzeugt werden. Der transmittierte UV-Lichtanteil wird an dem ersten Erfassungsort und damit an dem Ort erfasst, an welchem bereits der transmittierte Lichtanteil des zwecks der Blutdetektion eingestrahlten Lichts erfasst wird.

Der Schritt g) umfasst das Erfassen des an der Dialysatströmung vorbeigestrahlten UV-Lichts. Das UV-Licht wird direkt oder gegebenenfalls ein- oder mehrfach umgelenkt in Richtung des zweiten Erfassungsorts abgestrahlt und dort - ebenso wie der gestreute Lichtanteil des zwecks Blutdetektion eingestrahlten Lichts - erfasst. Der Schritt g) umfasst zudem das Erzeugen des vierten Signals. Das vierte Signal repräsentiert die Intensität des erfassten vorbeigestrahlten UV-Lichts und fungiert als Referenzsignal für das dritte Signal. Denn die Intensität des vorbeigestrahlten UV-Lichts ist unabhängig von der Harnstoffkonzentration. Somit können etwaige Schwankungen in der Helligkeit des emittierten UV-Lichts bei einer Auswertung des dritten und des vierten Signals als solche erkannt werden. Das vierte Signal kann zeitkontinuierlich und/oder zeitdiskret erzeugt werden.

Der Schritt h) umfasst das Ermitteln des Kt/V-Werts. Die Ermittlung erfolgt in Abhängigkeit des erzeugten dritten Signals und des erzeugten vierten Signals. Hierbei fungiert das vierte Signal als Referenzsignal. Das dritte Signal repräsentiert die Intensität des erfassten transmittierten UV-Lichtanteils, der wiederum mit der UV-Lichtabsorption in Zusammenhang steht. Bekanntermaßen besteht eine annähernd lineare Beziehung zwischen der UV-Lichtabsorption und der Harnstoffkonzentration in der Dialysatströmung, so dass der Kt/V-Wert auf der Grundlage prinzipiell bekannter Zusammenhänge ermittelt wird.

In weiterer Ausgestaltung der Erfindung wird der zweite Erfassungsort von dem in die Dialysatströmung eingestrahlten UV-Licht abgeschirmt. Hierdurch wird vermieden, dass das in die Dialysatströmung eingestrahlte UV-Licht direkt, reflektiert oder durch sonstige optische Effekte in Richtung des zweiten Erfassungsorts gelenkt wird und dort das Erfassen des vorbeigestrahlten UV-Lichts und somit das Erzeugen des vierten Signals in unerwünschter Weise verfälscht.

In weiterer Ausgestaltung der Erfindung werden das Licht und das UV-Licht abwechselnd, vorzugsweise mit einer Wechselfrequenz von 1 kHz, in die Dialysatströmung eingestrahlt, wobei das erste Signal und das dritte Signal abwechselnd mittels eines an dem ersten Erfassungsort angeordneten ersten Detektors erzeugt werden, und wobei das zweite Signal und das vierte Signal abwechselnd mittels eines an dem zweiten Erfassungsort angeordneten zweiten Detektors erzeugt werden. Dies ist eine besonders bevorzugte Ausgestaltung der Erfindung. Bei einer hinreichend hohen Wechselfrequenz sind eine quasi-gleichzeitige Blutleckdetektion und Kt/V-Wert-Ermittlung möglich.

Die erfindungsgemäße Detektionseinrichtung ist zum Durchführen des vorbeschriebenen Verfahrens eingerichtet und weist auf: wenigstens eine Lichtquelle, die zum Einstrahlen von Licht in die Dialysatströmung eingerichtet ist; einen ersten Detektor, der an einem ersten Erfassungsort angeordnet ist, wobei der erste Detektor zum Erfassen eines durch die Dialysatströmung hindurch transmittierten Lichtanteils des eingestrahlten Lichts und zum Erzeugen eines ersten Signals, das die Intensität des erfassten transmittierten Lichtanteils repräsentiert, eingerichtet ist; einen zweiten Detektor, der an einem von dem ersten Erfassungsort unterschiedlichen zweiten Erfassungsort angeordnet ist, wobei der zweite Detektor zum Erfassen eines in der Dialysatströmung gestreuten Lichtanteils des eingestrahlten Lichts und zum Erzeugen eines zweiten Signals eingerichtet ist, das die Intensität des erfassten gestreuten Lichtanteils repräsentiert; und aufweisend eine Auswerteeinheit, die zum Erzeugen eines Detektionssignals in Abhängigkeit des ersten Signals und des zweiten Signals eingerichtet ist. Durch die erfindungsgemäße Lösung kann insbesondere auf eine Kalibriereinrichtung zum Kalibrieren der Helligkeit der Lichtquelle verzichtet werden. Denn etwaige betriebsbedingte Schwankungen der Helligkeit der Lichtquelle bzw. des von dieser eingestrahlten Lichts werden gleichermaßen mittels des ersten Detektors und mittels des zweiten Detektors erfasst. Dies kann beim Erzeugen des Detektionssignals mittels der Auswerteeinheit entsprechend berücksichtigt werden. Im Ergebnis ermöglicht die erfindungsgemäße Detektionseinrichtung einen einfachen Aufbau und eine dennoch zuverlässige Detektion von Blut in der Dialysatströmung, da fehlerhafte Detektionen aufgrund von Helligkeitsschwankungen der Lichtquelle bei gleichzeitigem Verzicht auf eine Kalibriereinrichtung vermieden werden. Die Lichtquelle ist insbesondere zum Ausführen des Schritts a) des erfindungsgemäßen Verfahrens eingerichtet. Die Lichtquelle ist vorzugsweise eine Leuchtdiode. Weiter vorzugsweise ist die Lichtquelle zum Emittieren von rotem, grünem und/oder blauem Licht eingerichtet und insoweit beispielsweise eine rote, grüne und/oder blaue Leuchtdiode. Der erste Detektor ist insbesondere zum Ausführen des Schritts b) des erfindungsgemäßen Verfahrens eingerichtet. Vorzugsweise ist der erste Detektor eine Photodiode. Der zweite Detektor ist insbesondere zum Ausführen des Schritts c) des erfindungsgemäßen Verfahrens eingerichtet. Vorzugsweise ist der zweite Detektor eine Photodiode. Der erste Detektor und der zweite Detektor sind an unterschiedlichen Orten, nämlich dem ersten Erfassungsort und dem zweiten Erfassungsort angeordnet. Insoweit sind der erste Detektor und der zweite Detektor voneinander beabstandet. Die Auswerteeinheit ist insbesondere zum Ausführen des Schritts d) des erfindungsgemäßen Verfahrens eingerichtet. Zur Vermeidung von Wiederholungen wird im Übrigen auf die Erläuterung der Merkmale und Vorteile des erfindungsgemäßen Verfahrens verwiesen. Das dort Gesagte ist sinngemäß auf die Einrichtung der Detektionseinrichtung, insbesondere die Lichtquelle, den ersten Detektor, den zweiten Detektor und/oder die Auswerteeinheit übertragbar.

In weiterer Ausgestaltung der Erfindung ist die Auswerteeinheit zum Erzeugen des Detektionssignals in Abhängigkeit einer zeitlichen Änderung des ersten Signals und einer zeitlichen Änderung des zweiten Signals eingerichtet. Insoweit ist die Auswerteeinheit insbesondere zum Ausführen der Ausgestaltung des erfindungsgemäßen Verfahrens gemäß Anspruch 2 eingerichtet. Im Übrigen wird ergänzend und zur Vermeidung von Wiederholungen auf die Offenbarung im Zusammenhang mit der vorgenannten Ausgestaltung des erfindungsgemäßen Verfahrens verwiesen, die sinngemäß auf diese Ausgestaltung der erfindungsgemäßen Detektionseinrichtung übertragbar ist.

In weiterer Ausgestaltung der Erfindung ist vorgesehen, dass die Lichtquelle auf einer ersten Seite eines lichtdurchlässigen Fluidleitungskanals angeordnet ist, der zur Fluidleitung der Dialysatströmung entlang seiner Längsrichtung vorgesehen ist, dass der erste Detektor in einer Einstrahlrichtung des Lichts von der Lichtquelle beabstandet auf einer der ersten Seite quer zur Längsrichtung des Fluidleitungskanals gegenüberliegenden zweiten Seite des Fluidleitungskanals angeordnet ist, und dass der zweite Detektor auf der zweiten Seite des Fluidleitungskanals und senkrecht zu dessen Längsrichtung beabstandet von dem ersten Detektor angeordnet ist. Der Fluidleitungskanal ist vorzugsweise aus einem transparenten Kunststoff oder aus Glas gefertigt. Der Fluidleitungskanal kann insbesondere als Schlauchabschnitt, Rohrabschnitt und bevorzugt als Küvette gestaltet sein. Weiter vorzugsweise weist der Fluidleitungskanal einen kreisrunden Querschnitt auf. Die Fluidleitung durch den Fluidleitungskanal erfolgt in Längsrichtung des Fluidleitungskanals. Die Lichtquelle und der erste Detektor sind auf einander gegenüberliegenden Seiten, nämlich der ersten Seite bzw. der zweiten Seite, des Fluidleitungskanals angeordnet. Vorzugsweise sind die Lichtquelle und der erste Detektor in einer gemeinsamen Mittellängsebene und/oder Mittelquerebene des Fluidleitungskanals angeordnet. Die Einstrahlrichtung des Lichts ist insoweit quer zur Längsrichtung des Fluidleitungskanals und damit auch quer zur Strömungsrichtung der Dialysatströmung orientiert. Vorzugsweise ist eine senkrechte Orientierung, d.h. eine Orientierung der Einstrahlrichtung unter 90° zur Längsrichtung bzw. Strömungsrichtung, vorgesehen. Vorzugsweise ist der zweite Detektor - in Bezug auf eine zwischen der Lichtquelle und dem ersten Detektor direkt erstreckte optische Achse und in einer senkrecht auf einen Querschnitt des Fluidleitungskanals gerichteten Blickrichtung - relativ zu dem ersten Detektor nach oben oder unten versetzt angeordnet.

In weiterer Ausgestaltung der Erfindung ist der zweite Detektor unter Ausbildung eines Winkels zwischen 5° und 30°, bevorzugt zwischen 18° und 22°, zur Einstrahlrichtung des Lichts angeordnet. Die besagte direkt zwischen der Lichtquelle und dem ersten Detektor erstreckte optische Achse ist entlang der Einstrahlrichtung erstreckt. Insoweit ist der zweite Detektor unter Ausbildung des vorbeschriebenen Winkels zu der optischen Achse angeordnet. Ein Winkel zwischen 5° und 30° ermöglicht eine funktionsgerechte Erfassung des in der Dialysatströmung gestreuten Lichtanteils. Zudem haben die Erfinder erkannt, dass ein Winkel zwischen 18° und 22°, besonders bevorzugt 20°, zur Erfassung des gestreuten Lichtanteils besondere Vorteile bietet.

In weiterer Ausgestaltung der Erfindung ist vorgesehen, dass eine UV-Lichtquelle vorgesehen und zum Einstrahlen von UV-Licht in die Dialysatströmung und zum Vorbeistrahlen von UV-Licht an der Dialysatströmung eingerichtet ist; dass der erste Detektor zum Erfassen eines durch die Dialysatströmung hindurch transmittierten UV-Lichtanteils des eingestrahlten UV-Lichts und zum Erzeugen eines dritten Signals, das die Intensität des erfassten transmittierten UV-Lichtanteils repräsentiert, eingerichtet ist; dass der zweite Detektor zum Erfassen des an der Dialysatströmung vorbeigestrahlten UV-Lichts und zum Erzeugen eines vierten Signals, das die Intensität des erfassten vorbeigestrahlten UV-Lichtanteils repräsentiert, eingerichtet ist; und dass die Auswerteeinheit zum Ermitteln eines Kt/V-Werts in Abhängigkeit des erzeugten dritten Signals und des erzeugten vierten Signals eingerichtet ist. Diese Ausgestaltung der erfindungsgemäßen Detektionseinrichtung ermöglicht eine Durchführung des gemäß Anspruch 3 ausgestalteten erfindungsgemäßen Verfahrens. Zur Vermeidung von Wiederholungen wird auf die Offenbarung zu der besagten Ausgestaltung des erfindungsgemäßen Verfahrens verwiesen, wobei die dort erläuterten Merkmale und Vorteile sinngemäß auf diese Ausgestaltung der erfindungsgemäßen Detektionseinrichtung übertragbar sind. Die UV-Lichtquelle ist insbesondere zum Ausführen des Schritts e) des Verfahrens eingerichtet. Die UV-Lichtquelle ist vorzugsweise eine Leuchtdiode. Die UV-Lichtquelle ist relativ zu der Dialysatströmung und/oder dem Fluidleitungskanal derart angeordnet, dass das emittierte UV-Licht teilweise in die Dialysatströmung einstrahlbar und teilweise an derselben vorbeistrahlbar ist. Mit anderen Worten ausgedrückt ist die UV-Lichtquelle derart angeordnet, dass eine erste optische Achse zwischen der UV-Lichtquelle und dem ersten Detektor und damit dem ersten Erfassungsort durch die Dialysatströmung erstreckt ist und dass eine zweite optische Achse zwischen der UV-Lichtquelle - und abseits der Dialysatströmung und/oder dem Fluidleitungskanal - dem zweiten Detektor und damit dem zweiten Erfassungsort erstreckt ist. Der erste Detektor ist bei dieser Ausgestaltung der Erfindung zusätzlich zum Ausführen des Schritts f) des Verfahrens eingerichtet. Der zweite Detektor ist bei dieser Ausgestaltung der Erfindung zusätzlich zum Ausführen des Schritts g) des Verfahrens eingerichtet. Die Auswerteeinheit ist bei dieser Ausgestaltung der Erfindung zusätzlich zum Ausführen des Schritts h) des Verfahrens eingerichtet. Im Übrigen wird ergänzend und zur Vermeidung von Wiederholungen auf die Offenbarung in Zusammenhang mit den vorgenannten Schritten e) bis h) des Verfahrens nach Anspruch 3 verwiesen, die in sinngemäßer Weise auf die Einrichtung der UV-Lichtquelle, des ersten Detektors, des zweiten Detektors und/oder der Auswerteeinheit übertragbar ist.

In weiterer Ausgestaltung der Erfindung ist die UV-Lichtquelle auf der ersten Seite des Fluidleitungskanals angeordnet. Vorzugsweise ist die UV-Lichtquelle in einer gemeinsamen Ebene mit der Lichtquelle und dem ersten Detektor und/oder dem zweiten Detektor angeordnet. In weiterer Ausgestaltung der Erfindung ist ein Abschirmelement vorgesehen, mittels dessen der zweite Detektor von dem in die Dialysatströmung eingestrahlten UV-Licht abgeschirmt ist. Zur Vermeidung von Wiederholungen wird auf die Offenbarung zu der Ausgestaltung des erfindungsgemäßen Verfahrens nach Anspruch 4 verwiesen, wobei das dort Gesagte sinngemäß gilt. Das Abschirmelement ist derart angeordnet, dass das Erfassen des gestreuten Lichtanteils des eingestrahlten Lichts und das Erfassen des an der Dialysatströmung vorbeigestrahlten UV-Lichts mittels des zweiten Detektors nicht von dem Abschirmelement beeinträchtigt ist. Das Abschirmelement ist wenigstens für die Wellenlänge des emittierten UV-Lichts undurchlässig. Vorzugsweise ist das Abschirmelement im Wesentlichen vollständig lichtundurchlässig.

In weiterer Ausgestaltung der Erfindung ist eine Steuereinheit vorgesehen und zur abwechselnden Ansteuerung der Lichtquelle und der UV-Lichtquelle, vorzugsweise mit einer Wechselfrequenz von 1 kHz, eingerichtet. Durch das abwechselnde Ansteuern werden das Licht und das UV-Licht zeitlich abwechselnd und somit jeweils intermittierend abgegeben und damit auch dementsprechend abwechselnd mittels des ersten Detektors und des zweiten Detektors erfasst. Die Steuereinheit kann eine gesonderte Einheit der Detektionseinrichtung sein oder gemeinsam mit der Auswerteeinheit in eine einzige Einheit integriert sein.

In weiterer Ausgestaltung der Erfindung ist ein Gehäuse vorgesehen, in welchem wenigstens die Lichtquelle, der erste Detektor und der zweite Detektor aufgenommen sind. Diese Ausgestaltung der Erfindung erleichtert insbesondere eine Montage und Wartung der Detektionseinrichtung, da die in dem Gehäuse aufgenommenen Bauteile auf einfache Weise unter Handhabung des Gehäuses an dem Dialysegerät gemeinsam montiert und/oder gemeinsam von diesem demontiert werden können.

In weiterer Ausgestaltung der Erfindung ist die UV-Lichtquelle und/oder das Abschirmelement in dem Gehäuse aufgenommen. Dies ist eine besonders bevorzugte Ausgestaltung der Erfindung, da auf ein zusätzliches Gehäuse zur Aufnahme der UV-Lichtquelle und/oder des Abschirmelements verzichtet werden kann. Stattdessen sind die für die Blutleckdetektion und die für die Kt/V-Wert-Ermittlung notwendigen Bauteile, insbesondere optischen Elemente, gemeinsam in dem Gehäuse aufgenommen.

Die Erfindung betrifft zudem ein Dialysegerät mit einem Dialysator und einer auslassseitig des Dialysators angeordneten Detektionseinrichtung gemäß der vorstehenden Beschreibung.

Weitere Vorteile ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt eine schematische Darstellung eines Abschnitts einer Ausführungsform eines erfindungsgemäßen Dialysegeräts, das mit einer Ausführungsform einer erfindungsgemäßen Detektionseinrichtung versehen ist,
- Fig. 2: eine schematisch stark vereinfachte Darstellung der Detektionseinrichtung nach Fig. 1, wobei diese zum Durchführen einer Ausführungsform des erfindungsgemäßen Verfahrens eingerichtet ist,
- Fig. 3: in einer der Fig. 2 entsprechenden Darstellungsweise eine weitere Ausführungsform einer erfindungsgemäßen Detektionseinrichtung, wobei diese zum Durchführen einer weiteren Ausführungsform des erfindungsgemäßen Verfahrens eingerichtet ist,
- Fig. 4: ein schematisches Diagramm zur weiteren Verdeutlichung der Funktionsweise der Detektionseinrichtung nach Fig. 2 und des mit dieser durchführbaren Verfahrens,
- Fig. 5: ein weiteres schematisches Diagramm zur Verdeutlichung der Funktionsweise der Detektionseinrichtung nach Fig. 3 und des mit dieser durchführbaren Verfahrens,
- Fig. 6: ein schematisches Flussdiagramm zur weiteren Verdeutlichung des mit der Detektionseinrichtung nach Fig. 2 durchführbaren Verfahrens und
- Fig. 7: ein schematisches Flussdiagramm zur Verdeutlichung des mit der Detektionseinrichtung nach Fig. 3 durchführbaren Verfahrens.

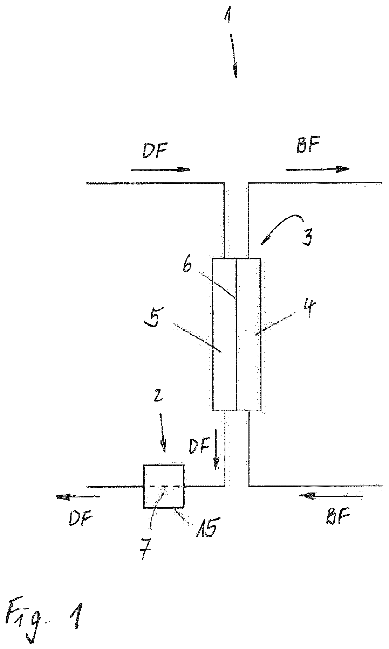

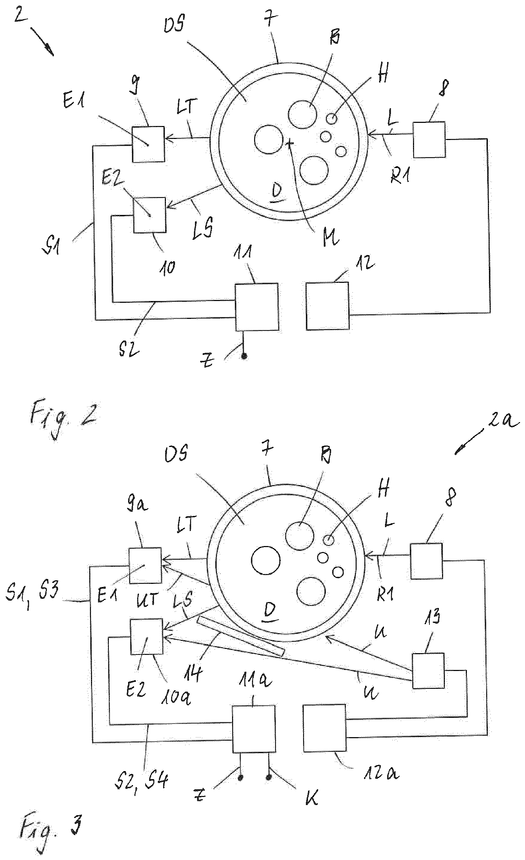

Fig. 1 zeigt schematisch einen Ausschnitt einer Ausführungsform eines erfindungsgemäßen Dialysegeräts 1, das mit einer Ausführungsform einer erfindungsgemäßen Detektionseinrichtung 2 versehen ist. Das Dialysegerät 1 ist zur extrakorporalen Blutbehandlung vorgesehen und weist einen Dialysator 3 mit einer Blutkammer 4 und einer Dialysatkammer 5 auf. Die Blutkammer 4 ist mittels einer semipermeablen Membran 6 von der Dialysatkammer 5 getrennt und fluidleitend an einen nicht näher bezeichneten extrakorporalen Blutkreislauf angeschlossen, in welchem zu behandelndes Blut entlang einer Fließrichtung BF durch die Blutkammer 4 gefördert wird. Die Dialysatkammer 5 ist an einen nicht näher bezeichneten Dialysatkreislauf angeschlossen, in welchem Dialysat D (Fig. 2), das auch als Dialysierflüssigkeit bezeichnet werden kann, unter Ausbildung einer Dialysatströmung DS entlang einer Fließrichtung DF durch die Dialysatkammer 5 gefördert wird.

Bei der extrakorporalen Blutbehandlung treten harnpflichtige Stoffe aus dem durch die Blutkammer 4 geförderten Blut über die semipermeable Membran 6 in die durch die Dialysatkammer 5 geförderte Dialysatströmung DS über. Diese harnpflichtigen Stoffe umfassen insbesondere Harnstoff H, der zwecks zeichnerischer Verdeutlichung anhand Fig. 2 schematisch stark vereinfachend teilchen- oder tropfenförmig in der Dialysatströmung DS dargestellt ist. In einem funktionstüchtigen Zustand der semipermeablen Membran 6 ist das Blut fluiddicht in der Blutkammer 4 gegenüber der Dialysatkammer 5 abgedichtet. Im Falle einer versagensbedingten Ruptur der semipermeablen Membran 6 leckt Blut von der Blutkammer 4 über die Ruptur in die Dialysatkammer 5 und damit in die Dialysatströmung DS ein. Zur zeichnerischen Verdeutlichung ist auf diese Weise in die Dialysatströmung DS eingelecktes Blut B schematisch stark vereinfachend teilchen- oder tropfenförmig dargestellt. Ein solches Blutleck kann zu schwerwiegenden medizinischen Komplikationen des zu behandelnden Patienten und zu technischen Beeinträchtigungen des Dialysegeräts 1 führen.

Die Detektionseinrichtung 2 dient zur Detektion des in die Dialysatströmung DS eingeleckten Bluts B. Zu diesem Zweck ist die Detektionseinrichtung 2 in Fließrichtung DF auslassseitig der Dialysatkammer 5 angeordnet und wird im betriebsfertig montierten Zustand von der Dialysatströmung DS durchflossen. Zur Fluidleitung der Dialysatströmung DS ist wenigstens im Bereich der Detektionseinrichtung 2 ein Fluidleitungskanal 7 vorgesehen. Der Fluidleitungskanal 7 ist bei der gezeigten Ausführungsform als ein Abschnitt des geräteseitigen Dialysatkreislaufs ausgebildet und insoweit nicht Bestandteil der Detektionseinrichtung 2. Bei einer nicht gezeigten Ausführungsform ist der Fluidleitungskanal stattdessen ein Bauteil der Detektionseinrichtung, das einlassseitig und auslassseitig der Detektionseinrichtung jeweils fluidleitend an dem geräteseitigen Dialysatkreislauf angeschlossen ist.

Wie anhand Fig. 2 gezeigt ist, weist die Detektionseinrichtung 2 eine Lichtquelle 8, einen ersten Detektor 9, einen zweiten Detektor 10 und eine Auswerteeinheit 11 auf.

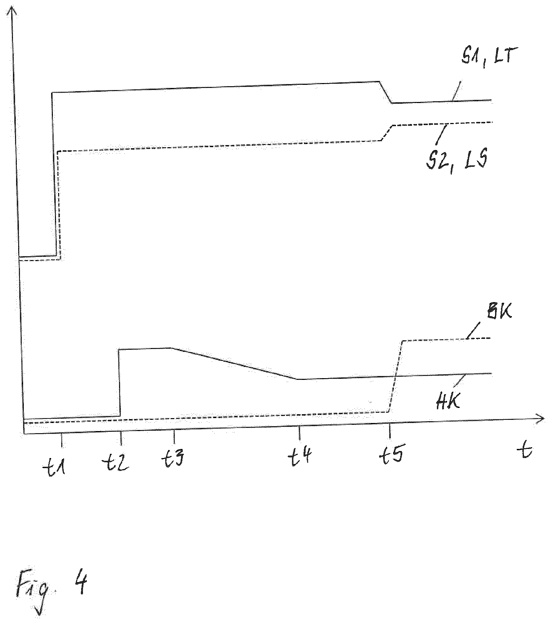

Die Lichtquelle 8 ist zum Einstrahlen von Licht L in die Dialysatströmung DS eingerichtet. Der erste Detektor 9 ist an einem ersten Erfassungsort E1 angeordnet, der anhand Fig. 2 vereinfachend im Zentrum der schematischen Darstellung des ersten Detektors 9 eingezeichnet ist. Der erste Detektor 9 ist zum Erfassen eines durch die Dialysatströmung DS transmittierten Lichtanteils LT des eingestrahlten Lichts L und zum Erzeugen eines ersten Signals S1 eingerichtet. Dabei repräsentiert das erste Signal S1 die Intensität des erfassten transmittierten Lichtanteils LT. Der zweite Detektor 10 ist an einem von dem ersten Erfassungsort E1 auf noch näher beschriebene Weise beabstandet angeordneten zweiten Erfassungsort E2 angeordnet. Dabei ist der zweite Detektor 10 zum Erfassen eines in der Dialysatströmung DS gestreuten Lichtanteils LS des eingestrahlten Lichts L und zum Erzeugen eines zweiten Signals S2 eingerichtet. Das zweite Signal S2 repräsentiert die Intensität des erfassten gestreuten Lichtanteils LS. Die Auswerteeinheit 11 ist zum Erzeugen eines Detektionssignals Z in Abhängigkeit des ersten Signals S1 und des zweiten Signals S2 eingerichtet. Genauer ist die Auswerteeinheit 11 bei der gezeigten Ausführungsform zum Erzeugen des Detektionssignals Z in Abhängigkeit einer anhand Fig. 4 noch näher zu erläuternden zeitlichen Änderung des ersten Signals S1 und einer zeitlichen Änderung des zweiten Signals S2 eingerichtet.

Zur Detektion von auf die vorbeschriebene Weise in die Dialysatströmung DS eingelecktem Blut B wird das Licht L mittels der Lichtquelle 8 durch den lichtdurchlässigen Fluidleitungskanal 7 in die Dialysatströmung DS eingestrahlt. Das eingestrahlte Licht L wird in der Dialysatströmung DS teilweise an dem in der Dialysatströmung DS befindlichen Blut B gestreut und von diesem absorbiert. Der sich hierbei ergebende transmittierte Lichtanteil LT wird am ersten Erfassungsort E1 mittels des ersten Detektors 9 erfasst und in das erste Signal S1 umgesetzt. Der sich hierbei ergebende gestreute Lichtanteil LS wird am zweiten Erfassungsort E2 mittels des zweiten Detektors 10 erfasst und in das zweite Signal S2 umgesetzt. Die Signale S1, S2 werden mittels der Auswerteeinheit 11 verarbeitet, die zu diesem Zweck mittels nicht näher bezeichneter Signalleitungen sowohl mit dem ersten Detektor 9 als auch mit dem zweiten Detektor 10 verbunden ist. Bei der gezeigten Ausführungsform wird das Detektionssignal Z dann erzeugt, wenn die zeitlichen Änderungen der Signale S1, S2 zueinander gegenläufig sind. Dies wird nachfolgend anhand Fig. 4 verdeutlicht.

Fig. 4 zeigt das erste Signal S1 und das zweite Signal S2 über einer Zeit t für einen exemplarischen Verlauf der extrakorporalen Blutbehandlung mittels des Dialysegeräts 1. Die exemplarisch gezeigten Verläufe der Signale S1, S2 repräsentieren, wie oben dargelegt, den erfassten transmittierten Lichtanteil LT bzw. den erfassten gestreuten Lichtanteil LS, so dass deren Verläufe deckungsgleich mit dem der Signale S1, S2 eingezeichnet sind. Zudem zeigt das Diagramm nach Fig. 4 den zeitlichen Verlauf einer Blutkonzentration BK des Bluts B und einer Harnstoffkonzentration HK des Harnstoffs H in der Dialysatströmung DS über der Zeit t.

Die exemplarische extrakorporale Blutbehandlung sieht zwischen den Zeitpunkten t1 und t2 ein sog. Priming, d.h. Entlüften, des Dialysatkreislaufs vor. Hierbei wird der Dialysatkreislauf mit dem Dialysat D befüllt, wobei insbesondere aus dem Fluidleitungskanal 7 Luft ausgespült und durch Dialysat D verdrängt wird. Infolge der unterschiedlichen optischen Eigenschaften von Luft und Dialysat D ändern sich naturgemäß die transmittierten und gestreuten Lichtanteile LT bzw. LS. Diese steigen zum Zeitpunkt t1 an. Entsprechendes gilt für die Signale S1, S2.

Zum Zeitpunkt t2 beginnt die eigentliche Blutbehandlung, bei welcher harnpflichtige Stoffe, insbesondere der Harnstoff H, über die semipermeable Membran 6 aus der Blutkammer 4 in die Dialysatströmung DS übertreten. Dementsprechend steigt die Harnstoffkonzentration HK zum Zeitpunkt t2 an. Eine Veränderung des transmittierten Lichtanteils LT und/oder des gestreuten Lichtanteils LS wird durch die steigende Harnstoffkonzentration HK nicht bewirkt.

Die Harnstoffkonzentration HK bleibt bis zum Zeitpunkt t3 unverändert. Sodann setzt eine Abnahme der Harnstoffkonzentration HK ein, die sich bis zum Zeitpunkt t4 fortsetzt. Auch diese zeitliche Änderung der Harnstoffkonzentration HK zeigt keine Auswirkung auf die Signale S1, S2 und/oder die Lichtanteile LT, LS. D.h. der Lichtanteil LT und damit auch das erste Signal S1 bleiben hiervon unberührt. Auch der Lichtanteil LS und somit das zweite Signal S2 bleiben hiervon unberührt.

Ab dem Zeitpunkt t4 findet keine weitere Veränderung der Harnstoffkonzentration HK statt.

Zum Zeitpunkt t5 tritt eine Ruptur an der semipermeablen Membran 6 auf, so dass Blut aus der Blutkammer 4 in die Dialysatkammer 5 und damit in die Dialysatströmung DS übertritt. Dies führt zu einem Anstieg der Blutkonzentration BK. Die zunehmende Blutkonzentration BK führt zu einer verstärkten Streuung des eingestrahlten Lichts L an dem eingeleckten Blut B (Fig. 2). Dementsprechend nimmt der gestreute Lichtanteil LS zum Zeitpunkt t5 zu. Durch diese Zunahme des gestreuten Lichtanteils LS kommt es zu einer entsprechenden zeitlichen Änderung des zweiten Signals S2. Gleichzeitig kommt es zu einer gegenläufigen zeitlichen Änderung des transmittierten Lichtanteils LT und damit auch des ersten Signals S1.

Die vorbeschriebene gegenläufige zeitliche Änderung der Lichtanteile LT, LS und damit auch des ersten Signals S1 und des zweiten Signals S2 ist ein eindeutiger Indikator für das zum Zeitpunkt t5 auftretende Blutleck BK. Dementsprechend wird das Detektionssignal Z mittels der Auswerteeinheit 11 dann ausgegeben, wenn eine solche vorbeschriebene zeitlich gegenläufige Änderung der Signale S1, S2 vorliegt.

Das Detektionssignal Z ist bei der vorliegenden Ausführungsform ein von einem Anwender des Dialysatgeräts 1 wahrnehmbares akustisches und/oder optisches Warnsignal. Bei einer nicht gezeigten Ausführungsform ist das Detektionssignal Z ein Steuersignal zur Steuerung wenigstens einer Funktion des Dialysegeräts 1. Beispielsweise kann das Dialysegerät 1 mittels des Detektionssignals Z zu einem Abbruch der extrakorporalen Blutbehandlung angesteuert werden, bei dem die Förderung des Bluts innerhalb des Blutkreislaufs und/oder des Dialysats D in dem Dialysatkreislauf unterbrochen wird.

Im Übrigen versteht sich, dass die vorliegend exemplarisch zum Zeitpunkt t5 auftretende Ruptur stattdessen selbstverständlich zu beliebigen anderen Zeitpunkten der extrakorporalen Blutbehandlung auftreten kann, beispielweise vor dem Zeitpunkt t4.

Bei der gezeigten Ausführungsform ist die Lichtquelle 8 auf einer nicht näher bezeichneten ersten Seite des lichtdurchlässigen Fluidleitungskanals 7 angeordnet. Sowohl der erste Detektor 9 als auch der zweite Detektor 10 sind auf einer zweiten Seite des Fluidleitungskanals 7 angeordnet, die der ersten Seite und damit auch der Lichtquelle 8 quer, genauer senkrecht, zur Fließrichtung DF der Dialysatströmung DS gegenüberliegt. Die Fließrichtung DF ist in Bezug auf die Zeichenebene der Fig. 2 senkrecht aus der Bildebene herausragend orientiert. Mit anderen Worten ausgedrückt ist der erste Detektor 9 entlang einer Einstrahlrichtung R1 des Lichts L von der Lichtquelle 8 beabstandet angeordnet.

Bei der vorliegenden Ausführungsform sind die Lichtquelle 8 und der erste Detektor 9 jeweils auf Höhe einer nicht näher bezeichneten Mittelquerachse des Fluidleitungskanals 7 angeordnet. Eine gedachte optische Achse zwischen der Lichtquelle 8 und dem ersten Detektor 9 und damit auch dem ersten Erfassungsort E1 ist somit koaxial zu der Mittelquerachse des Fluidleitungskanals 7 ausgerichtet.

Der zweite Detektor 10 ist vorliegend in Bezug auf die Mittelquerachse des Fluidleitungskanals 7 versetzt angeordnet. Dabei ist der zweite Detektor 10 unter Ausbildung eines nicht näher bezeichneten Winkels zur Einstrahlrichtung R1 des Lichts L angeordnet. Mit anderen Worten ausgedrückt ist der zweite Detektor 10 in einem noch näher spezifizierten Winkel zu einem Mittelpunkt M des Fluidleitungskanals 7 positioniert. Anders als Fig. 2 vermuten lässt beträgt der Winkel zur Einstrahlrichtung R1 und damit auch zur Mittelquerachse des Fluidleitungskanals 7 vorliegend 20°.

Die Lichtquelle 8, der erste Detektor 9 und der zweite Detektor 10 sind bei der gezeigten Ausführungsform in einer gemeinsamen Ebene angeordnet.

Bei der Ausführungsform nach Fig. 2 ist die Lichtquelle 8 eine Leuchtdiode. Zu deren Ansteuerung ist vorliegend eine Steuereinrichtung 12 vorgesehen. Die Lichtquelle 8 ist mittels der Steuereinrichtung 12 zur zeitkontinuierlichen und/oder zeitdiskreten, d.h. intermittierenden, Emission des Lichts L ansteuerbar. Der erste Detektor 9 und der zweite Detektor 10 sind jeweils eine Photodiode. Die Auswerteeinheit 11 und die Steuereinheit 12 können - wie in Fig. 2 schematisch angedeutet - als baulich und/oder funktional getrennte Einheiten vorgesehen sein. Bei einer nicht gezeigten Ausführungsform sind die Auswerteeinheit und die Steuereinheit in eine gemeinsame Einheit integriert.

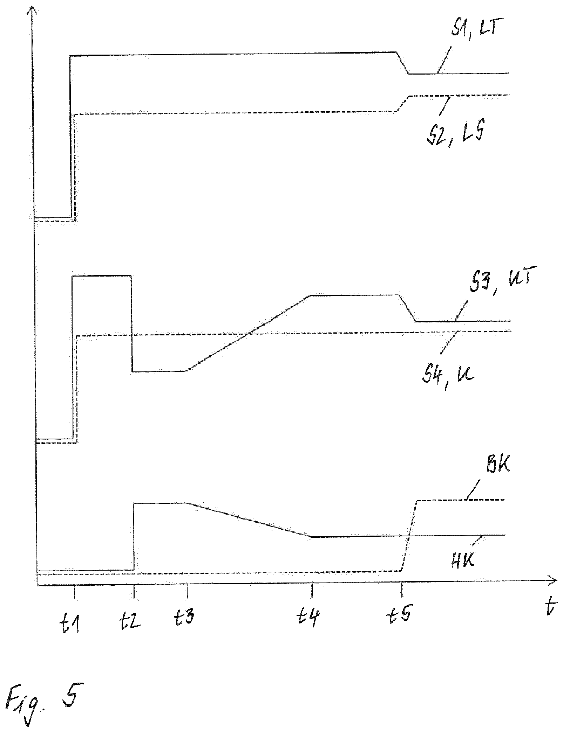

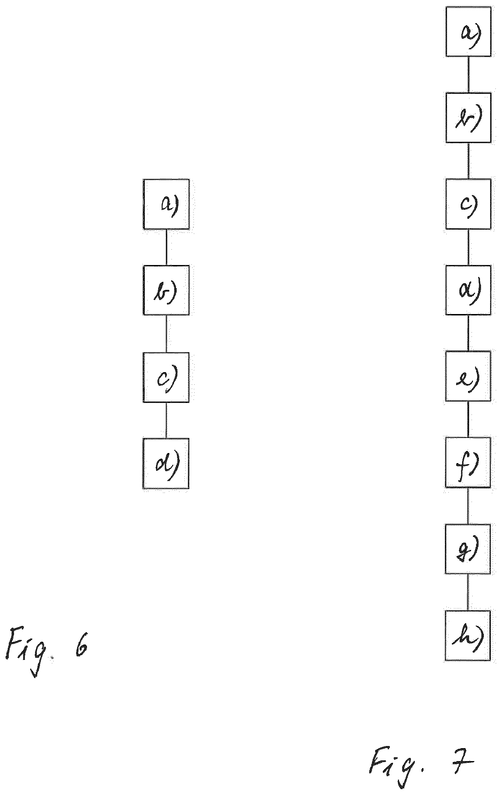

Die Detektionseinrichtung 2a nach Fig. 3 weist einen weitgehend übereinstimmenden Aufbau mit der Detektionseinrichtung 2 nach Fig. 2 auf. Zur Vermeidung von Wiederholungen wird auf die diesbezügliche Offenbarung im Zusammenhang mit der Detektionseinrichtung 2 nach Fig. 2 verwiesen, die sinngemäß auch im Hinblick auf die Detektionseinrichtung 2a gilt. Nachfolgend wird lediglich auf wesentliche Unterschiede der Detektionseinrichtung 2a eingegangen. Aufgrund dieser Unterschiede ist die Detektionseinrichtung 2a zum Durchführen des anhand der Fig. 5 und 7 schematisch verdeutlichten Verfahrens zur Detektion von Blut und Harnstoff in der Dialysatströmung DS eingerichtet.

Die Detektionseinrichtung 2a unterscheidet sich im Wesentlichen dadurch von der Detektionseinrichtung 2, dass eine UV-Lichtquelle 13 vorgesehen ist. Die UV-Lichtquelle 13 ist zum Einstrahlen von UV-Licht U in die Dialysatströmung DS und zum Vorbeistrahlen von UV-Licht U an der Dialysatströmung DS eingerichtet. Der erste Detektor 9a ist entsprechend dem ersten Detektor 9 der Detektionseinrichtung 2 nach Fig. 2 eingerichtet. Zusätzlich ist der erste Detektor 9a zum Erfassen eines durch die Dialysatströmung DS hindurch transmittierten UV-Lichtanteils UT des eingestrahlten UV-Lichts U und zum Erzeugen eines dritten Signals S3 eingerichtet. Das dritte Signal S3 repräsentiert die Intensität des erfassten transmittierten UV-Lichtanteils UT. Der zweite Detektor 10a ist entsprechend dem zweiten Detektor 10 der Detektionseinrichtung 2 nach Fig. 2 eingerichtet. Zusätzlich ist der zweite Detektor 10a zum Erfassen des an der Dialysatströmung DS vorbeigestrahlten UV-Lichts U und zum Erzeugen eines vierten Signals S4 eingerichtet. Das vierte Signal S4 repräsentiert die Intensität des erfassten vorbeigestrahlten UV-Lichts U. Die Auswerteeinheit 11a ist gemäß der Auswerteeinheit 11 der Detektionseinrichtung 2 nach Fig. 2 eingerichtet. Zusätzlich ist die Auswerteeinheit 11a zum Ermitteln eines Kt/V-Werts K in Abhängigkeit des erzeugten dritten Signals S3 und des erzeugten vierten Signals S4 eingerichtet.

Der Kt/V-Wert K ist eine im Bereich der Dialysetechnik grundsätzlich bekannte Größe und lässt einen Rückschluss auf den Fortschritt der extrakorporalen Blutbehandlung zu. Der Kt/V-Wert K wird bekanntlich auf Grundlage der Harnstoffkonzentration HK des Harnstoffs H in der Dialysatströmung DS ermittelt. Die Harnstoffkonzentration HK steht bekanntlich in einer annähernd linearen Beziehung mit der Absorption des in die Dialysatströmung DS eingestrahlten UV-Lichts U. Dementsprechend ändert sich der transmittierte UV-Lichtanteil UT in Abhängigkeit der Harnstoffkonzentration HK. Eine solche Änderung wird mittels des ersten Detektors 9a erfasst und in das dritte Signal S3 umgesetzt. Dabei dient die Erfassung des an der Dialysatströmung DS vorbeigestrahlten UV-Lichts U mittels des zweiten Detektors 10a und dessen Umsetzung in das vierte Signal S4 als Referenz. Die mittels der Auswerteeinheit 11a auszuführenden Auswerteoperationen zur Ermittlung des Kt/V-Werts K in Abhängigkeit des dritten Signals S3 und des vierten Signals S4 sind als solche grundsätzlich bekannt, so dass von weiteren diesbezüglichen Erläuterungen abgesehen werden kann.

Fig. 5 zeigt in einer zu Fig. 4 entsprechenden Weise einen exemplarischen Verlauf der extrakorporalen Blutbehandlung mittels des Dialysegeräts 1 über der Zeit t und unter Verwendung der Detektionseinrichtung 2a. Dabei entsprechen die dort gezeigten exemplarischen Verläufe der Signale S1, S2 bzw. die entsprechenden Verläufe des transmittierten Lichtanteils LT und des erfassten gestreuten Lichtanteils LS den bereits anhand Fig. 4 erläuterten Verläufen. Entsprechendes gilt hinsichtlich der Harnstoffkonzentration HK und der Blutkonzentration BK. Zur Vermeidung von Wiederholungen wird daher auf die diesbezüglichen Erläuterungen im Zusammenhang mit Fig. 4 verwiesen.

Fig. 5 zeigt zusätzlich das dritte Signal S3 und das vierte Signal S4 über der Zeit. Die exemplarisch gezeigten Verläufe der Signale S3, S4 repräsentieren, wie oben dargelegt, die Intensität des erfassten transmittierten UV-Lichtanteils UT bzw. die Intensität des erfassten vorbeigestrahlten UV-Lichts U.

Mit dem Ansteigen der Harnstoffkonzentration HK zum Zeitpunkt t2 tritt eine verstärkte Extinktion des in die Dialysatströmung DS eingestrahlten UV-Lichts ein, so dass der transmittierte UV-Lichtanteil UT und dementsprechend auch das dritte Signal S3 sinkt. Die Intensität des erfassten vorbeigestrahlten UV-Lichts U und somit auch das vierte Signal S4 bleiben hiervon unberührt.

Mit der zum Zeitpunkt t3 einsetzenden Abnahme der Harnstoffkonzentration HK steigt der transmittierte UV-Lichtanteil UT und damit auch das dritte Signal S3 an. Dies bis zum Zeitpunkt t4. Ab dem Zeitpunkt t4 bleibt die Harnstoffkonzentration HK unverändert.

Aufgrund des zum Zeitpunkt t5 auftretenden Blutlecks kommt es zudem zu einer Abnahme des transmittierten UV-Lichtanteils UT und damit auch des dritten Signals S3. Diese Änderung des dritten Signals S3 zeigt keine praktische Auswirkung auf die Ermittlung des Kt/V-Werts K, da die extrakorporale Blutbehandlung nach der Detektion des zum Zeitpunkt t5 auftretenden Blutlecks ohnehin unterbrochen wird.

Die UV-Lichtquelle 13 ist bei der gezeigten Ausführungsform in einer gemeinsamen Ebene mit dem ersten Detektor 9a und dem zweiten Detektor 10a angeordnet. Somit liegen vorliegend sämtliche optischen Bauelemente 8, 9a, 10a, 13 in einer gemeinsamen Ebene. Die UV-Lichtquelle 13 ist relativ zu dem Fluidleitungskanal 7 und der Lichtquelle 8 nach unten versetzt angeordnet, so dass eine direkte Abstrahlung des UV-Lichts von der UV-Lichtquelle 13 in Richtung des zweiten Erfassungsorts E2 und damit auch das zweiten Detektors 10a erfolgen kann. Diese Abstrahlung erfolgt insoweit an dem Fluidleitungskanal 7 vorbei.

Die Detektionseinrichtung 2a weist zudem ein Abschirmelement 14 auf, das relativ zu dem Fluidleitungskanal 7, der UV-Lichtquelle 13 und/oder dem zweiten Detektor 10a derart angeordnet ist, dass letzterer mittels des Abschirmelements 14 von in der Dialysatströmung DS gestreuten oder anderweitig in Richtung des zweiten Erfassungsorts E2 abgelenkten Lichtanteilen des eingestrahlten UV-Lichts U abgeschirmt ist. Gleichzeitig ist das Abschirmelement 14 derart angeordnet, dass der in der Dialysatströmung DS gestreute Lichtanteil LS des eingestrahlten Lichts L mittels des zweiten Detektors 10a erfassbar ist.

Die Steuereinrichtung 12a ist bei der Ausführungsform nach Fig. 3 zum abwechselnden Ansteuern der Lichtquelle 8 und der UV-Lichtquelle 13 eingerichtet. Die Ansteuerung erfolgt vorzugsweise mit einer Wechselfrequenz von 1 kHz. Mit anderen Worten ausgedrückt werden das Licht L und das UV-Licht U zeitlich abwechselnd in die Dialysatströmung DS eingestrahlt und dementsprechend zeitlich abwechselnd mittels des ersten Detektors 9a und des zweiten Detektors 10a in die Signale S1, S2 bzw. S3, S4 umgesetzt. Bei einer hinreichend hohen Wechselfrequenz erfolgen eine in praktischer Hinsicht quasi-gleichzeitige Erzeugung des Detektionssignals Z und Ermittlung des Kt/V-Werts K.

Wie weiter anhand Fig. 1 gezeigt ist, ist ein Gehäuse 15 vorgesehen. Das Gehäuse 15 nimmt bei der Ausführungsform nach Fig. 2 wenigstens die Lichtquelle 8, den ersten Detektor 9 und den zweiten Detektor 10 auf. Zusätzlich können die Auswerteeinheit 11 und die Steuereinheit 12 in dem Gehäuse 15 aufgenommen sein.

Entsprechendes gilt im Hinblick auf die Ausführungsform nach Fig. 3, so dass auch dort ein Gehäuse zur Aufnahme im Wesentlichen sämtlicher Bauteile der Detektionseinrichtung 2a vorgesehen sein kann.

Fig. 6 zeigt eine schematisch stark vereinfachte Flussdiagrammdarstellung des mittels der Detektionseinrichtung 2 nach Fig. 2 durchführbaren Verfahrens zur Detektion von Blut umfassend die Schritte a) bis d). Hierzu ergänzend wird auf den Wortlaut von Anspruch 1 verwiesen. Fig. 7 zeigt ein schematisch stark vereinfachtes Flussdiagramm des mit der Detektionseinrichtung 2a nach Fig. 3 durchführbaren Verfahrens zur Detektion von Blut und Harnstoff umfassend die Schritte a) bis h). Hierzu ergänzend wird auf den Wortlaut der Ansprüche 1 und 3 Bezug genommen.

## Patentansprüche

1. Verfahren zur Detektion von Blut (B) in einer Dialysatströmung (DS) eines Dialysegeräts (1) bei einer extrakorporalen Blutbehandlung, das folgende Schritte umfasst:
a) Einstrahlen von Licht (L) in die Dialysatströmung (DS);
b) Erfassen eines durch die Dialysatströmung (DS) hindurch transmittierten Lichtanteils (LT) des eingestrahlten Lichts (L) an einem ersten Erfassungsort (E1) und Erzeugen eines ersten Signals (S1), das die Intensität des erfassten transmittierten Lichtanteils (LT) repräsentiert;
c) Erfassen eines in der Dialysatströmung (DS) gestreuten Lichtanteils (LS) des eingestrahlten Lichts (L) an einem zweiten Erfassungsort (E2) und Erzeugen eines zweiten Signals (S2), das die Intensität des erfassten gestreuten Lichtanteils (LS) repräsentiert;
d) Erzeugen eines Detektionssignals (Z) in Abhängigkeit des erzeugten ersten Signals (S1) und des erzeugten zweiten Signals (S2);
e) Emittieren von UV-Licht (U), wobei das UV-Licht (U) in die Dialysatströmung (DS) eingestrahlt und an der Dialysatströmung (DS) vorbeigestrahlt wird;
f) Erfassen eines durch die Dialysatströmung (DS) hindurch transmittierten UV-Lichtanteils (UT) des eingestrahlten UV-Lichts (U) an dem ersten Erfassungsort (E1) und Erzeugen eines dritten Signals (S3), das die Intensität des erfassten transmittierten UV-Lichtanteils (UT) repräsentiert;
g) Erfassen des an der Dialysatströmung (DS) vorbeigestrahlten UV-Lichts (U) an dem zweiten Erfassungsort (E2) und Erzeugen eines vierten Signals (S4), das die Intensität des erfassten vorbeigestrahlten UV-Lichts (U) repräsentiert;
h) Ermitteln eines Kt/V-Werts (K) in Abhängigkeit des erzeugten dritten Signals (S3) und des erzeugten vierten Signals (S4).

2. Verfahren nach Anspruch 1, wobei das Detektionssignal (Z) dann erzeugt wird, wenn eine zeitliche Änderung des ersten Signals (S1) gegenläufig ist zu einer zeitlichen Änderung des zweiten Signals (S2).

3. Verfahren nach Anspruch 1 oder 2, wobei der zweite Erfassungsort (E2) von dem in die Dialysatströmung (DS) eingestrahlten UV-Licht (U) abgeschirmt wird.

4. Verfahren nach einem der vorhergehenden Ansprüche, wobei das Licht (L) und das UV-Licht (U) abwechselnd in die Dialysatströmung (DS) eingestrahlt werden, wobei das erste Signal (S1) und das dritte Signal (S3) abwechselnd mittels eines an dem ersten Erfassungsort (E1) angeordneten ersten Detektors (9a) erzeugt werden, und wobei das zweite Signal (S2) und das vierte Signal (S4) abwechselnd mittels eines an dem zweiten Erfassungsort (E2) angeordneten zweiten Detektors (10a) erzeugt werden.

5. Detektionseinrichtung (2, 2a) zum Durchführen eines Verfahrens nach einem der vorhergehenden Ansprüche, aufweisend
- wenigstens eine Lichtquelle (8), die zum Einstrahlen von Licht (L) in die Dialysatströmung (DS) eingerichtet ist;
- einen ersten Detektor (9, 9a), der an einem ersten Erfassungsort (E1) angeordnet ist, wobei der erste Detektor (9, 9a) zum Erfassen eines durch die Dialysatströmung (DS) hindurch transmittierten Lichtanteils (LT) des eingestrahlten Lichts (L) und zum Erzeugen eines ersten Signals (S1), das die Intensität des erfassten transmittierten Lichtanteils (LT) repräsentiert, eingerichtet ist;
- einen zweiten Detektor (10, 10a), der an einem von dem ersten Erfassungsort (E1) unterschiedlichen zweiten Erfassungsort (E2) angeordnet ist, wobei der zweite Detektor (10, 10a) zum Erfassen eines in der Dialysatströmung (DS) gestreuten Lichtanteils (LS) des eingestrahlten Lichts (L) und zum Erzeugen eines zweiten Signals (S2) eingerichtet ist, das die Intensität des erfassten gestreuten Lichtanteils (LS) repräsentiert;
- eine Auswerteeinheit (11, 11a), die zum Erzeugen eines Detektionssignals (Z) in Abhängigkeit des ersten Signals (S1) und des zweiten Signals (S2) eingerichtet ist; und
- aufweisend eine UV-Lichtquelle (13), die zum Einstrahlen von UV-Licht (U) in die Dialysatströmung (DS) und zum Vorbeistrahlen von UV-Licht (U) an der Dialysatströmung (DS) eingerichtet ist;
- wobei der erste Detektor (9a) zum Erfassen eines durch die Dialysatströmung (DS) hindurch transmittierten UV-Lichtanteils (UT) des eingestrahlten UV-Lichts (U) und zum Erzeugen eines dritten Signals (S3), das die Intensität des erfassten transmittierten UV-Lichtanteils (UT) repräsentiert, eingerichtet ist;
- wobei der zweite Detektor (10a) zum Erfassen des an der Dialysatströmung (DS) vorbeigestrahlten UV-Lichts (U) und zum Erzeugen eines vierten Signals (S4), das die Intensität des erfassten vorbeigestrahlten UV-Lichts (U) repräsentiert, eingerichtet ist;
- und wobei die Auswerteeinheit (11a) zum Ermitteln eines Kt/V-Werts (K) in Abhängigkeit des erzeugten dritten Signals (S3) und des erzeugten vierten Signals (S4) eingerichtet ist.

6. Detektionseinrichtung (2, 2a) nach Anspruch 5, **dadurch gekennzeichnet, dass** die Auswerteeinheit (11, 11a) zum Erzeugen des Detektionssignals (Z) in Abhängigkeit einer zeitlichen Änderung des ersten Signals (S1) und einer zeitlichen Änderung des zweiten Signals (S2) eingerichtet ist.

7. Detektionseinrichtung (2, 2a) nach Anspruch 5 oder 6, **dadurch gekennzeichnet, dass** die Lichtquelle (8) auf einer ersten Seite eines lichtdurchlässigen Fluidleitungskanals (7) angeordnet ist, der zur Fluidleitung der Dialysatströmung (DS) entlang seiner Längsrichtung vorgesehen ist, dass der erste Detektor (9, 9a) in einer Einstrahlrichtung (R1) des Lichts (L) von der Lichtquelle (8) beabstandet auf einer der ersten Seite quer zur Längsrichtung des Fluidleitungskanals (7) gegenüberliegenden zweiten Seite des Fluidleitungskanals (7) angeordnet ist, und dass der zweite Detektor (10, 10a) auf der zweiten Seite des Fluidleitungskanals (7) und senkrecht zu dessen Längsrichtung beabstandet von dem ersten Detektor (9, 9a) angeordnet ist.

8. Detektionseinrichtung (2, 2a) nach Anspruch 7, **dadurch gekennzeichnet, dass** der zweite Detektor (10, 10a) unter Ausbildung eines Winkels zwischen 5° und 30° zur Einstrahlrichtung (R1) des Lichts angeordnet ist.

9. Detektionseinrichtung (2a) nach einem der Ansprüche 5 bis 8, **dadurch gekennzeichnet, dass** die UV-Lichtquelle (13) auf der ersten Seite des Fluidleitungskanals (7) angeordnet ist.

10. Detektionseinrichtung (2a) nach einem der Ansprüche 5 bis 9, **dadurch gekennzeichnet, dass** ein Abschirmelement (14) vorgesehen ist, mittels dessen der zweite Detektor (10a) von dem in die Dialysatströmung (DS) eingestrahlten UV-Licht (U) abgeschirmt ist.

11. Detektionseinrichtung (2a) nach einem der Ansprüche 5 bis 10, **dadurch gekennzeichnet, dass** eine Steuereinheit (12a) vorgesehen und zur abwechselnden Ansteuerung der Lichtquelle (8) und der UV-Lichtquelle (13) eingerichtet ist.

12. Detektionseinrichtung (2, 2a) nach einem der Ansprüche 5 bis 11, **dadurch gekennzeichnet, dass** ein Gehäuse (15) vorgesehen ist, in welchem wenigstens die Lichtquelle (8), der erste Detektor (9, 9a) und der zweite Detektor (10, 10a) aufgenommen sind.

13. Detektionseinrichtung (2a) nach Anspruch 12, **dadurch gekennzeichnet, dass** die UV-Lichtquelle (13) und/oder das Abschirmelement (14) in dem Gehäuse (15) aufgenommen sind.

14. Dialysegerät (1) mit einem Dialysator (3) und einer auslassseitig des Dialysators (3) angeordneten Detektionseinrichtung (2, 2a) nach einem der vorhergehenden Ansprüche.

## Claims

1. Method for detecting blood (B) in a dialysate flow (DS) of a dialysis machine (1) during an extracorporeal blood treatment, the method comprising the following steps:
a) radiating light (L) into the dialysate flow (DS);
b) registering, at a first detection location (E1), a light component (LT) of the light (L) radiated in that has been transmitted through the dialysate flow (DS) and producing a first signal (S1) which represents the intensity of the registered transmitted light component (LT) ;
c) registering, at a second detection location (E2), a light component (LS) of the light (L) radiated in that has been scattered in the dialysate flow (DS) and producing a second signal (S2) which represents the intensity of the registered scattered light component (LS) ;
d) producing a detection signal (Z) on the basis of the first signal (S1) produced and the second signal (S2) produced;
e) emitting UV light (U), with the UV light (U) being radiated into the dialysate flow (DS) and being radiated past the dialysate flow (DS);
f) registering, at the first detection location (E1), a UV light component (UT) of the UV light (U) radiated in that has been transmitted through the dialysate flow (DS) and producing a third signal (S3) which represents the intensity of the registered transmitted UV light component (UT);
g) registering, at the second detection location (E2), the UV light (U) radiated past the dialysate flow (DS) and producing a fourth signal (S4) which represents the intensity of the registered UV light (U) radiated past said dialysate flow;
h) determining a Kt/V value (K) on the basis of the third signal (S3) produced and the fourth signal (S4) produced.

2. Method according to Claim 1, wherein the detection signal (Z) is produced when a change in the first signal (S1) over time is in the opposite sense to a change in the second signal (S2) over time.

3. Method according to Claim 1 or 2, wherein the second detection location (E2) is shielded from the UV light (U) radiated into the dialysate flow (DS).

4. Method according to any one of the preceding claims, wherein the light (L) and the UV light (U) are radiated into the dialysate flow (DS) in alternating fashion, with the first signal (S1) and the third signal (S3) being produced alternately by means of a first detector (9a) arranged at the first detection location (E1) and with the second signal (S2) and the fourth signal (S4) being produced alternately by means of a second detector (10a) arranged at the second detection location (E2).

5. Detection device (2, 2a) for carrying out a method according to any one of the preceding claims, comprising
- at least one light source (8) configured to radiate light (L) into the dialysate flow (DS);
- a first detector (9, 9a) arranged at a first detection location (E1), with the first detector (9, 9a) being configured to register a light component (LT) of the light (L) radiated in that has been transmitted through the dialysate flow (DS) and produce a first signal (S1) which represents the intensity of the registered transmitted light component (LT);
- a second detector (10, 10a) arranged at a second detection location (E2) that differs from the first detection location (E1), with the second detector (10, 10a) being configured to register a light component (LS) of the light (L) radiated in that has been scattered in the dialysate flow (DS) and produce a second signal (S2) which represents the intensity of the registered scattered light component (LS);
- an evaluation unit (11, 11a) configured to produce a detection signal (Z) on the basis of the first signal (S1) and the second signal (S2); and
- comprising a UV light source (13) configured to radiate UV light (U) into the dialysate flow (DS) and radiate UV light (U) past the dialysate flow (DS);
- with the first detector (9a) being configured to register a UV light component (UT) of the UV light (U) radiated in that has been transmitted through the dialysate flow (DS) and produce a third signal (S3) which represents the intensity of the registered transmitted UV light component (UT);
- with the second detector (10a) being configured to register the UV light (U) radiated past the dialysate flow (DS) and produce a fourth signal (S4) which represents the intensity of the registered UV light (U) radiated past said dialysate flow;
- and with the evaluation unit (11a) being configured to determine a Kt/V value (K) on the basis of the third signal (S3) produced and the fourth signal (S4) produced.

6. Detection device (2, 2a) according to Claim 5, **characterized in that** the evaluation unit (11, 11a) is configured to produce the detection signal (Z) on the basis of a change in the first signal (S1) over time and a change in the second signal (S2) over time.

7. Detection device (2, 2a) according to Claim 5 or 6, **characterized in that** the light source (8) is arranged on a first side of a light-transmissive fluid-guiding channel (7) provided for fluid guidance of the dialysate flow (DS) along its longitudinal direction, **in that** the first detector (9, 9a) is arranged at a distance from the light source (8) in a radiated-in direction (R1) of the light (L) on a second side of the fluid-guiding channel (7) which transversely to the longitudinal direction of the fluid-guiding channel (7) is located opposite the first side, and **in that** the second detector (10, 10a) is arranged on the second side of the fluid-guiding channel (7) and at a distance from the first detector (9, 9a) perpendicular to the longitudinal direction of said fluid-guiding channel.

8. Detection device (2, 2a) according to Claim 7, **characterized in that** the second detector (10, 10a) is arranged so as form an angle of between 5° and 30° with respect to the radiated-in direction (R1) of the light.

9. Detection device (2a) according to any one of Claims 5 to 8, **characterized in that** the UV light source (13) is arranged on the first side of the fluid-guiding channel (7).

10. Detection device (2a) according to any one of Claims 5 to 9, **characterized in that** a shielding element (14) is provided, by means of which the second detector (10a) is shielded from the UV light (U) radiated into the dialysate flow (DS).

11. Detection device (2a) according to any one of Claims 5 to 10, **characterized in that** a control unit (12a) is provided and configured to drive the light source (8) and the UV light source (13) alternately.

12. Detection device (2, 2a) according to any one of Claims 5 to 11, **characterized in that** a housing (15) is provided, in which at least the light source (8), the first detector (9, 9a) and the second detector (10, 10a) are received.

13. Detection device (2a) according to Claim 12, **characterized in that** the UV light source (13) and/or the shielding element (14) are received within the housing (15) .

14. Dialysis machine (1) comprising a dialyser (3) and a detection device (2, 2a) according to any one of the preceding claims arranged on the outlet-side of the dialyser (3).

## Revendications

1. Procédé de détection de sang (B) dans un flux de dialysat (DS) d'un appareil de dialyse (1) lors d'un traitement extracorporel du sang, lequel comprend les étapes suivantes :
a) irradiation de lumière (L) dans le flux de dialysat (DS) ;
b) détection d'une part de lumière (LT) de la lumière irradiée (L) transmise à travers le flux de dialysat (DS) au niveau d'un premier point de détection (E1) et génération d'un premier signal (S1) qui représente l'intensité de la part de lumière (LT) transmise détectée ;
c) détection d'une part de lumière (LS) de la lumière irradiée (L) diffusée dans le flux de dialysat (DS) au niveau d'un deuxième point de détection (E2) et génération d'un deuxième signal (S2) qui représente l'intensité de la part de lumière (LS) diffusée détectée ;
d) génération d'un signal de détection (Z) en fonction du premier signal (S1) détecté et du deuxième signal (S2) détecté ;
e) émission de lumière UV (U), la lumière UV (U) étant irradiée dans le flux de dialysat (DS) et étant rayonnée au voisinage du flux de dialysat (DS) ;
f) détection d'une part de lumière UV (UT) de la lumière UV (U) irradiée transmise à travers le flux de dialysat (DS) au niveau du premier point de détection (E1) et génération d'un troisième signal (S3) qui représente l'intensité de la part de lumière UV (UT) transmise détectée ;
g) détection de la lumière UV (U) rayonnée au voisinage du flux de dialysat (DS) au niveau du deuxième point de détection (E2) et génération d'un quatrième signal (S4) qui représente l'intensité de la lumière UV (U) rayonnée au voisinage détectée ;
h) détermination d'une valeur Kt/V (K) en fonction du troisième signal (S3) généré et du quatrième signal (S4) généré.

2. Procédé selon la revendication 1, le signal de détection (Z) étant généré lorsqu'une variation dans le temps du premier signal (S1) est en sens inverse d'une variation dans le temps du deuxième signal (S2).

3. Procédé selon la revendication 1 ou 2, le deuxième point de détection (E2) étant protégé par blindage de la lumière UV (U) irradiée dans le flux de dialysat (DS).

4. Procédé selon l'une des revendications précédentes, la lumière (L) et la lumière UV (U) étant irradiées en alternance dans le flux de dialysat (DS), le premier signal (S1) et le troisième signal (S3) étant générés en alternance au moyen d'un premier détecteur (9a) disposé au niveau du premier point de détection (E1), et le deuxième signal (S2) et le quatrième signal (S4) étant générés en alternance au moyen d'un deuxième détecteur (10a) disposé au niveau du deuxième point de détection (E2) .

5. Dispositif de détection (2, 2a) destiné à mettre en œuvre un procédé selon l'une des revendications précédentes, comprenant
- au moins une source de lumière (8), qui est conçue pour irradier de la lumière (L) dans le flux de dialysat (DS) ;
- un premier détecteur (9, 9a), qui est disposé au niveau d'un premier point de détection (E1), le premier détecteur (9, 9a) étant conçu pour détecter une part de lumière (LT) de la lumière irradiée (L) transmise à travers le flux de dialysat (DS) et pour générer un premier signal (S1) qui représente l'intensité de la part de lumière (LT) transmise détectée ;
- un deuxième détecteur (10, 10a), qui est disposé au niveau d'un deuxième point de détection (E2) différent du premier point de détection (E1), le deuxième détecteur (10, 10a) étant conçu pour détecter une part de lumière (LS) de la lumière irradiée (L) diffusée dans le flux de dialysat (DS) et pour générer un deuxième signal (S2) qui représente l'intensité de la part de lumière (LS) diffusée détectée ;
- une unité d'interprétation (11, 11a), qui est conçue pour générer un signal de détection (Z) en fonction du premier signal (S1) et du deuxième signal (S2) ; et
- comprenant une source de lumière UV (13), qui est conçue pour irradier de la lumière UV (U) dans le flux de dialysat (DS) et pour rayonnée de la lumière UV (U) au voisinage du flux de dialysat (DS) ;
- le premier détecteur (9a) étant conçu pour détecter une part de lumière UV (UT) de la lumière UV (U) irradiée transmise à travers le flux de dialysat (DS) et pour générer un troisième signal (S3) qui représente l'intensité de la part de lumière UV (UT) transmise détectée ;
- le deuxième détecteur (10a) étant conçu pour détecter la lumière UV (U) rayonnée au voisinage du flux de dialysat (DS) et pour générer un quatrième signal (S4) qui représente l'intensité de la lumière UV (U) rayonnée au voisinage détectée ;
- et l'unité d'interprétation (11a) étant conçue pour déterminer une valeur Kt/V (K) en fonction du troisième signal (S3) généré et du quatrième signal (S4) généré.

6. Dispositif de détection (2, 2a) selon la revendication 5, **caractérisé en ce que** l'unité d'interprétation (11, 11a) est conçue pour générer le signal de détection (Z) en fonction d'une variation dans le temps du premier signal (S1) et d'une variation dans le temps du deuxième signal (S2).

7. Dispositif de détection (2, 2a) selon la revendication 5 ou 6, **caractérisé en ce que** la source de lumière (8) est disposée sur un premier côté d'un conduit de fluide (7) transparent, lequel est destiné à la conduction fluidique du flux de dialysat (DS) le long de sa direction longitudinale, **en ce que** le premier détecteur (9, 9a) est disposé espacé de la source de lumière (8) dans une direction d'irradiation (R1) de la lumière (L) sur un deuxième côté du conduit de fluide (7) qui se trouve à l'opposé du premier côté transversalement à la direction longitudinale du conduit de fluide (7), et **en ce que** le deuxième détecteur (10, 10a) est disposé sur le deuxième côté du conduit de fluide (7) et espacé du premier détecteur (9, 9a) perpendiculairement à sa direction longitudinale.

8. Dispositif de détection (2, 2a) selon la revendication 7, **caractérisé en ce que** le deuxième détecteur (10, 10a) est disposé en formant un angle entre 5° et 30° par rapport à la direction d'irradiation (R1) de la lumière.

9. Dispositif de détection (2a) selon l'une des revendications 5 à 8, **caractérisé en ce que** la source de lumière UV (13) est disposée sur le premier côté du conduit de fluide (7).

10. Dispositif de détection (2a) selon l'une des revendications 5 à 9, **caractérisé en ce qu'**un élément de protection par blindage (14) est présent, au moyen duquel le deuxième détecteur (10a) est protégé par blindage de la lumière UV (U) irradiée dans le flux de dialysat (DS).

11. Dispositif de détection (2a) selon l'une des revendications 5 à 10, **caractérisé en ce qu'**une unité de commande (12a) est présente et conçue pour commander en alternance la source de lumière (8) et la source de lumière UV (13).

12. Dispositif de détection (2, 2a) selon l'une des revendications 5 à 11, **caractérisé en ce qu'**un boîtier (15) est présent, dans lequel sont logés au moins la source de lumière (8), le premier détecteur (9, 9a) et le deuxième détecteur (10, 10a).

13. Dispositif de détection (2a) selon la revendication 12, **caractérisé en ce que** la source de lumière UV (13) et/ou l'élément de protection par blindage (14) sont logés dans le boîtier (15).

14. Appareil de dialyse (1) comprenant un dialyseur (3) et un dispositif de détection (2, 2a) selon l'une des revendications précédentes qui est disposé du côté de la sortie du dialyseur (3).
